(19) Europäisches Patentamt
European Patent Office
Office européen des brevets

(11) Veröffentlichungsnummer: **0 166 971**
**B1**

(12) **EUROPÄISCHE PATENTSCHRIFT**

(45) Veröffentlichungstag der Patentschrift: 28.02.90

(51) Int. Cl.$^5$: **A 61 K 37/26**

(21) Anmeldenummer: 85106669.6

(22) Anmeldetag: 30.05.85

(54) **Insulinzubereitungen, Verfahren zu deren Herstellung und deren Verwendung.**

(30) Priorität: 09.06.84 DE 3421613
09.06.84 DE 3421615
01.12.84 DE 3443877

(43) Veröffentlichungstag der Anmeldung:
08.01.86 Patentblatt 86/02

(45) Bekanntmachung des Hinweises auf die Patenterteilung:
28.02.90 Patentblatt 90/09

(84) Bennante Vertragsstaaten:
AT BE CH DE FR GB IT LI LU NL SE

(56) Entgegenhaltungen:
GB-A-2 101 482
US-A-2 474 729
US-A-2 574 889

CHEMICAL ABSTRACTS, Band 77, Nr. 9, 28. August 1972, Seite 73, Nr. 56944t, Columbus, Ohio, US; SUZUKI, FUJIO et al.: "Mode of action of insulin. Properties and biological activity of an insulin-dextran complex" & ENDOCRINOLOGY 1972, 90 (5), 1220-30

Diabetologia(1980),S.1,5,7,8.

(73) Patentinhaber: HOECHST AKTIENGESELLSCHAFT
Postfach 80 03 20
D-6230 Frankfurt am Main 80 (DE)

(72) Erfinder: Grau, Ulrich, Dr.
Zeil 17
D-6238 Hofheim am Taunus (DE)
Erfinder: Ross, Gerhard, Dr.
Friedensstrasse 22
D-6238 Hofheim am Taunus (DE)
Erfinder: Hiller, Dietrich, Dr.
Riederbergstrasse 11
D-6200 Wiesbaden (DE)
Erfinder: Neubauer, Horst Paul, Dr.
Am Eichkopf 12
D-6240 Königstein/Taunus (DE)

EP 0 166 971 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung bezieht sich auf die Herstellung von neuartigen Insulinzubereitungen mit verzögerter Wirkung, die Herstellung von stabilisierten Insulinzubereitungen für den Einsatz in automatischen Dosiergeräten, sowie deren Verwendung, insbesondere zur Behandlung des Diabetes mellitus.

Es ist allgemein bekannt, daß an die parenterale Substitutionstherapie mit Insulin spezielle Anforderungen gestellt werden. Dazu zählt insbesondere die Frage einer verzögerten Pharmakokinetik, die es erlaubt, den Diabetiker mit einer oder einigen wenigen Injektionen am Tag einzustellen. Zur Erzielung solcher Depoteffekte existieren einige klinisch bewährte Prinzipien, darunter die Verwendung von Zink oder Protaminsulfat als Depothilfsstoffe.

Diese bekannten Depotprinzipien beruhen auf dem physikalischen Effekt der langsamen Wiederauflösung einer bei physiologischem pH-Wert schwerlöslich vorliegenden Form des Insulins, z. B. in Form des 2-Zink-Kristalls. Weist dabei das Präparat bereits einen neutralen pH auf, was hinsichtlich der chemischen Stabilität bei langer Lagerung vorteilhaft ist, so sind diese Präparate Suspensionen, die vor der Dosierung sehr sorgfältig homogen aufgeschüttelt werden müssen, um Fehldosierungen zu vermeiden.

Die bisher bekannten Insulin-Depotpräparate weisen darüberhinaus sehr spezifische und nur innerhalb gewisser Grenzen durch Zusatz von gelöstem Insulin variierbare Wirkprofile auf. Es gibt nun immer Patienten, für die alternative Wirkprofile, z. B. solche mit etwas weniger schnellem Einsetzen bei etwa gleich langer Wirkung, wünschenswert sind. Hat der Arzt solche Präparate zur Verfügung, so kann er auf die spezifischen Gewohnheiten und Eigenheiten des Diabetikers eingehen. Würde man hingegen vom Patienten die Umstellung seiner Gewohnheiten fordern, so würde dieses das Problem der Patienten-"Compliance" mit sich bringen, die letztlich wesentlich den Therapieerfolg beeinflußt.

Kraegen et al. berichten in Brit. Med. J. 1975, *3*, 464 - 466 von einem stabilisierenden Effekt eines Zusatzes von bis zu 3,5 % Haemaccel zu sehr verdünnten Insulinlösungen (0,04 I.E./ml), wodurch sich unter anderem die Adsorption des Insulins am Vorratsgefäß und Schlauchsystem in Infusionsystemen verhindern läßt.

Es wurde nun gefunden, daß eine wäßrige Insulinzubereitung mit einer Insulinkonzentration von über 1 I.E./ml und einem Gehalt an stabilisierenden physiologisch verträglichen Mitteln überraschenderweise eine synergistisch verbesserte physikalische Stabilität und andere verbesserte Eigenschaften, z. B. hinsichtlich des Wirkprofils aufweist, wenn sie bei 4°C eine Viskosität von mindestens 1,75 mPa.s hat und als stabilisierende physiologisch verträgliche Mittel eine Kombination

A) eines physiologisch verträglichen Verdickungsmittels der Art Polygeline und/oder Dextran mit
B) einer physiologisch verträglichen oberflächenaktiven Substanz aus der Gruppe
B1) Äthylenglycol-Propylenglycol-Mischpolymerisat oder Blockpolymerisat, wobei jedoch mindestens die Hälfte der Kettenglieder die Einheiten des 1,2-Propylenglycols sind,
B2) Lecithin
und/oder
B3) Polyoxyäthylen-23-lauryläther
enthält.

**Diese Zubereitung ist gegen mechanische Belastung, insbesondere bei erhöhter Temperatur, z. B Schütteln und Pumpbewegung besonders gut stabilisiert und eignet sich daher u.a. speziell für den Einsatz in Peristaltikpumpen.**

Die Frage der Stabilität von Insulinzubereitungen ist schon bisher ein schwieriges Problem gewesen. So ist bekannt, daß gelöste Proteine wie Insulin an Grenzflächen (dazu gehört auch die Grenzfläche wäßrige Lösung/Luft) adsorbiert werden (C.W.N Cumber und A.E. Alexander, Trans. Faraday Soc. *46*, 235 (1950)). Als Folge dieser Adsorption an Grenzflächen werden verschiedene Sekundärreaktionen beobachtet, die man allgemein unter dem Begriff "Denaturierung" zusammenfaßt. Es kommt zu einer Formänderung der adsorbierten Proteinmoleküle (Änderung der Tertiär- und/oder Sekundärstruktur). Daneben kann es zur Aggregation von adsorbierten Molekülen zu löslichen oder unlöslichen polymeren Formen kommen. Auch die bei der Passage von Insulinlösungen durch enge Kanäle auftretende Turbulenz scheint eine Insulin-Denaturierung zu begünstigen.

Als ein Haupthindernis bei der Weiterentwicklung und der klinischen Anwendung kontinuierlicher Infusionsvorrichtungen hat sich die Neigung des Insulins, aus handelsüblichen Lösungen auszufallen und dadurch mechanische Teile sowie Versorgungswege zu verstopfen, erwiesen. Weiterhin gibt es die Tendenz, die Größe dieser Vorrichtungen zu reduzierten, um so implantierbare Systeme zu erhalten, wodurch sich ein Bedarf an höher konzentrierten, stabilen Insulinlösungen ergibt, was wiederum die obengenannten Probleme noch schwerwiegender macht.

Die Frage der physikalischen Stabilität von Insulinlösungen wird insbesondere seit Entwicklung von automatischen Dosiergeräten diskutiert. Es ist allgemein bekannt, daß in solchen Geräten speziell stabilisierte Insuline verwendet werden müsser.. Im Zusammenhang mit ungenügender physikalischer Stabilität von Insulinen wird nicht nur eine reduzierte biologische Wirksamkeit, sondern neuerdings auch ein über Stimulation der Makrophagen ablaufender Prozeß der Bildung von Amyloid-A-Protein im Serum, das zur Amyloidose in verschiedenen Organen führen kann, diskutiert (Brownlee et al., Lancet (1984), 411 - 413).

Zur Lösung dieser Probleme ist schon eine Reihe von Vorschlägen gemacht worden, wobei jedoch nirgends die Kombination von Verdickungsmittel + oberflächenaktive Substanz als Stabilisierungsmittel beschrieben ist: Hauptsächlich zwecks Erzielung einer verzögerten Wirkung wurden Insulinzubereitungen bereits verschiedene Verdickungsmittel zugesetzt (US-A-2 474 729: Polyvinylpyrrolidon; US-A-2 574 885: methylierte bzw. alkylierte Gelatine; C.A. Vol 77, 1972 ref. 56944 t: Dextran).

Ein Bestandteil der festen Insulinzubereitung gemäß CB-A-2 101 482 ist ein wasserlösliches Copolymer aus N-Vinylpyrrolidon, Acrylamid und Ethylacrylat; als Stabilisator dient dort ein spezielles Salicylsäurederivat.

Eine Zusammenstellung verschiedener nieder- und auch höhermolekularer Verbindungen, welche sich zur Stabilisierung von Insulin eignen, findet sich in dem Artikel von W.D. Lougheed et al. "Insulin Aggregation in Artificial Delivery Systems" in Diabetologia 19, 1 - 9 (1980).

Aus der DE-A-2 917 535 sind wäßrige Insulinlösungen bekannt, die zum Schutz gegen Denaturierung eine oberflächenaktive Substanz der allgemeinen Formel I

$$R^{b}O-(-CH_2-\overset{\overset{R^{a}}{|}}{C}H-O-)_{p}-R^{c} \qquad (I)$$

in der

$R^a$ Wasserstoff, Methyl oder Äthyl,

p die Zahl 2 - 80, vorzugsweise 8 - 45, bedeuten und

$R^b$ und $R^c$ gleich oder verschieden sind und Wasserstoff, Alkylalkoholreste mit 1 - 20 C-Atomen, Carbonsäurereste mit 2 - 20 C-Atomen, Alkylphenolreste mit einer Alkylkette von 1 - 10 C-Atomen oder Alkylaminreste mit 1 - 20 C-Atomen bedeuten, als Homopolymerisat, Blockpolymerisat oder Mischpolymerisat in einer Konzentration von 2 bis 200 mg/l enthalten.

In einer der EP-A-18 609 werden gegen Denaturierung beständige wäßrige Lösungen von Insulin und einer Vielzahl anderer Proteine beschrieben, die gekennzeichnet sind durch einen Gehalt an einer oberflächenaktiven Substanz mit kettenförmiger Grundstruktur, deren Glieder schwach hydrophobe und schwach hydrophile Bereiche in alternierender Anordnung enthalten.

Die WO-A-83/00 288 beschreibt stabile wäßrige Insulinzubereitungen zur Anwendung in Insulindosiervorrichtungen, die einen pH-Wert von 6,5 bis 9 aufweisen und die bis zu 1000 ppm eines Polyoxyäthylenalkyläthers der Formel $R^7$-O-$[CH_2$-$CH_2$-$O]_m$-H (II) enthalten, worin $R^7$ eine gesättige oder ungesättigte ($C_8$-$C_{15}$)-Alkylgruppe und m eine ganze Zahl von 2 bis 25 bedeuten.

Aus der DE-A-3 240 177 sind schließlich physikalisch stabilisierte Insulinlösungen bekannt, die dadurch gekennzeichnet sind, daß sie stabilisierende Mengen an einem Phospholipid der Formel III enthalten

$$\begin{array}{l} H-CH-OR^{4} \\ \quad | \\ \quad CH-OR^{5} \quad O \\ \quad | \qquad\quad // \\ H-CH-O-P-OR^{6} \\ \qquad\qquad\quad \backslash \\ \qquad\qquad\quad OH \end{array} \qquad (III)$$

in der

$R^4$ und $R^5$, die gleich oder verschieden sein können, für Wasserstoff, Alkylcarbonyl, Alkenylcarbonyl, Alkandienylcarbonyl, Alkantrienylcarbonyl oder Alkantetraenylcarbonyl stehen, mit der Maßgabe daß $R^4$ und $R^5$ nicht gleichzeitig Wasserstoff sind, und in der

$R^6$ für eine hydrophile Gruppe steht.

Diese grenzflächenaktiven Stabilisatoren sind äußerst effektiv, indem sie die Schüttelstabilität von Insulinlösungen deutlich erhöhen. Unzweifelhaft ist das Schütteln ein wesentlicher negativer Einfluß auf das Insulin in Dosiergeräten.

Nun hat sich aber gezeigt, daß insbesondere in Peristaltikpumpen das Quetschen des Elastomer-Pumpschlauchs und/oder Schereffekte, wie sie bei vielen Pumpprinzipien auftreten, zusätzlich die Insulinstabilität beeinträchtigen. Auf diese Weise kann es trotz Zusatz der verschiedenen bisher bekannten Stabilisatoren zu Insulinpräzipitation in Pumpschläuchen oder Kathetern kommen.

Unter "Insulinen" werden hier wie im folgenden einheitliche Produkte oder Gemische mehrerer Insuline, und zwar nicht nur Humaninsulin und Insuline tierischen Ursprungs, wie Säugerinsuline (beispielsweise aus Rind oder Schwein) verstanden; man versteht darunter auch Insuline im weiteren Sinn, d.h. modifizierte Insuline, wie des-$Phe^{B1}$-Insuline (vgl. z. B. DE-C-2 005 658, EP-A-46 979) oder am C-Terminus der B-Kette basisch modifizierte Insuline (wie Insulin-B31-Arg-OH oder Insulin-B31-Arg-Arg-OH, vorgeschlagen in den deutschen Offenligungsschriften DE-A-3 326 472, -3 327 709, -3 333 640 und -3 334 407) und humanes oder andere Proinsuline oder Proinsulinanaloge (vgl. z. B. DE-A-3 232 036) sowie die Alkali- und Ammoniumsalze. Es können auch mehrere dieser Insuline im Gemisch vorliegen. Die Insulinkonzentration kann, abhängig von der Löslichkeit, bis etwa 1500 I.E./ml betragen, vorzugsweise liegt sie zwischen 5 und etwa 1000 I.E./ml. In Depotformen kann ein beliebiger Anteil eines oder mehrerer Insuline unabhängig voneinander jeweils in gelöster, amorpher und/oder kristalliner Form vorliegen.

Als Verdickungsmittel (auch Geliermittel genannt) kommen speziell Polygeline (= Gelantinepartialhydrolysate, die auch, z. B. mit Diisocyanaten, vernetzt sein können) und/oder Dextran(e) in Frage, als oberflächenaktive

Substanzen Äthylenglycol-Propylenglycol-Mischpolymerisate oder Blockpolymerisate, wobei mindestens die Hälfte der Kettenglieder die Einheiten des 1,2-Propylenglycol sind, sowie Leicithin und/oder Polyoxyäthylen-23-lauryläther.

Die Verdickungsmittel bewirken, daß die Insulinzubereitung bei niedrigen Temperaturen, z. B. 4°C leicht dickflüssig bis dickflüssig oder als Hydrogel vorliegt. Die erfindungsgemäßen Zubereitungen haben, gemessen bei 4°C, vorzugsweise eine Viskosität von mindestens 2 und insbesondere eine solche von mindestens 2,5 mPa.s. Die Gele verflüssigen sich z. T. schon bei Raumtemperatur oder bei Temperaturen nahe Körpertemperatur.

Die erfindungsgemäße Zubereitung, die eine hohe physikalische Stabilität hat, enthält vorzugsweise mehr als 1, insbesondere zwischen 2 und 20 Gew.-% Verdickungsmittel. Doch können auch beim Zusatz geringerer Mengen Verdickungsmittel schon Hydrogele entstehen. Nach oben hin kann der Gehalt an Geliermittel, abhängig von dessen Art, 30 % und mehr betragen.

Bei genügend hohem Anteil an Verdickungsmittel ist allen Ausführungsformen der erfindungsgemäßen Zubereitung gemeinsam, daß sie unter Lagerungsbedingungen in fester Form als Hydrogel vorliegen. Dies ist ein Vorteil in Bezug auf die physikalische Stabilität, da bekanntlich Oligomerenbildung und Denaturierung durch Bewegung stark beschleunigt werden, also prinzipiell beim Handhaben von flüssigen Insulinzubereitungen nicht sicher ausgeschlossen werden können. Die Lagerung in Gelform kann auch deshalb vorteilhaft sein, weil diese Form bei der Lagerung wesentlich homogener bleibt als eine Lösung bzw. Suspension. Es ist z. B. bei sedimentierten Kristallsuspensionen bei langer Lagerung durchaus denkbar, daß sich relativ stabile Kristallassoziate bilden, die dann weniger leicht zu einer homogenen Suspension aufgeschüttelt werden können, so daß Dosierungsfehler eintreten können. Liegt dagegen die Kristallsuspension homogen in einem Gel "eingefroren" vor, so können die Insulinmoleküle nur langsam an die Gefäßwand oder die Grenzfläche Flüssigkeit/Luft diffundieren, und es sind solche Effekte auszuschließen. Außerdem sind Bewegungen und Turbulenzen innerhalb der Gele während der Handhabung erheblich reduziert. Die Zubereitung weist daher eine besonders gute Lagerstabilität auf.

Vor der Anwendung werden die Gele, wie auch bei konventionellen Insulinen üblich, auf Raum- bis Körpertemperatur gebracht, wobei sie sich verflüssigen, aber trotzdem noch eine gegenüber konventionellen Insulinlösungen erhöhte Viskosität aufweisen. Normalerweise werden dann Suspensionen nicht sofort sedimentieren, so daß Inhomogenitäten und Dosierungsfehler weniger leicht auftreten können; ein Inhomogenitätsproblem existiert selbstverständlich bei klaren Gelzubereitungen nicht. In jedem Fall lassen sich die gebräuchlichen Injektionsgeräte verwenden.

Die erhöhte physikalische Stabilität der erfindungsgemäßen Gele ist auch noch bei Körpertemperatur, also in flüssigem Zustand, zu einem gewissen Maß vorhanden. Werden solche Lösungen in einem Rotationsversuch bei 37°C thermisch-mechanisch belastet, so läßt sich eine relative Stabilität von ca. 3 bis 5 gegenüber konventionellen Insulinlösungen beobachten, die kein Verdickungsmittel enthalten.

Alle erfindungsgemäßen Zubereitungen lassen sich prinzipiell aus gelöstem oder aus amorphem oder kristallinem Insulin herstellen (klare bzw. trübe Gele). Sie weisen im allgemeinen einen pH-Wert zwischen 2,5 und 8,5, insbesondere aber zwischen 6 und 8 auf, enthalten vorzugsweise ein geeignetes Isotoniemittel, ein geeignetes Konservierungsmittel und gegebenenfalls eine geeignete Puffersubstanz, z. B. die weiter unten genannten. Der Wirkstoff liegt vorzugsweise gelöst vor.

Die erfindungsgemäße Zubereitung weist - wohl wegen des Gehalts an Verdickungsmittel - einen gewissen Depoteffekt auf.

Es ist möglich, den Depoteffekt durch Kombination mit geläufigen Hilfsmitteln mit verzögernder Wirkung, wie etwa durch Zugabe geeigneter Mengen von Zink, Surfen, Globin oder Protaminsulfat, zu verstärken. Die zugesetzte Zinkmenge kann dabei bis zu 100 µg $Zn^{2+}$/100 Insulineinheiten betragen; bevorzugt liegt sie über 35 und meistens unter 50 µg $Zn^{2+}$/100 Insulineinheiten. Die Protaminmenge kann z. B. zwischen 0,28 mg und 0,6 mg pro 100 Einheiten betragen (bezogen auf Protaminsulfat). Auf diese Weise sind bisher nicht zugängliche, besonders lang wirksame Präparationen herstellbar, deren Anwendung interessant ist, weil nach neueren Erkenntnissen aus der Therapie mit Insulindosiergeräten gerade eine Basalmenge an Insulin therapeutisch vorteilhaft erscheint.

Als physiologisch unbedenkliches und mit den Insulinen verträgliches Trägermedium eignet sich eine sterile wäßrige Lösung, die zu Blut in der üblichen Weise, z. B. durch Glycerin, Kochsalz, Glucose, isotonisch gemacht wird und daneben noch eines oder mehrerer der gebräuchlichen Konservierungsmittel, z. B. Phenol, m-Kresol, Benylalkohol oder p-Hydroxybenzoesäureester, enthält. Das Trägermedium kann zusätzlich eine Puffersubstanz, z.B. Natriumacetat, Natriumcitrat, Natriumphosphat, Tris-(hydroxymethyl)-aminomethan, enthalten. Zur Einstellung des pH werden verdünnte Säuren (typischerweise HCl) bzw. Laugen (typischerweise NaOH) verwendet.

Die erfindungsgemäße Insulinzubereitung wird dadurch hergestellt, daß man einer wäßrigen Insulinzubereitung ein physiologisch verträgliches Verdickungsmittel und eine physiologisch verträgliche oberflächenaktive Substanz der vorher genannten Art zusetzt. Die Zubereitungen zeichnen sich durch besondere Stabilität und - bei subkutaner oder intramuskulärer Gabe durch eine verzögerte Wirkung aus.

Ferner betrifft die Erfindung die Verwendung dieser Insulinzubereitungen bei der Behandlung des Diabetes mellitus, insbesondere mittels Vorrichtungen zur kontinuierlichen Insulinabgabe sowie die Verwendung der Zubereitungen zur Vermeidung der Adsorption oder Denaturierung von Insulin an Oberflächen und anderen Phasengrenzflächen, insbesondere bei der Reinigung durch Chromatographie oder Kristallisation, bei Lagerung und bei der therapeutischen Anwendung.

Die erfindungsgemäßen Insulinzubereitungen können zur Behandlung des Diabetes mellitus parenteral, d.h. intravenös, subkutan oder intramuskulär appliziert werden. Der Depoteffekt der Zubereitungen mit oder ohne

oberflächenaktive Substanzen zeigt sich am ausgeprägtesten bei der subkutanen Applikationsweise, ist aber auch deutlich bei intramuskulärer Injektion. Intravasal appliziert wirken die erfindungsgemäßen klar gelösten Zubereitungen rasch wie die bekannten gelösten Insuline. Sie sind daher hervorragend geeignet zur Verwendung in automatischen Dosiergeräten, wie Pumpen, bei denen das infundierte Insulin sofort wirksam sein muß, da nur so eine rasche Steuerung, z. B. entsprechend dem Blutglucosespiegel, möglich ist.

Bei einigen Dosierprinzipien ist es nötig, oder zumindest vorteilhaft, entgaste Insulinlösung in das Reservoir einzufüllen. Luft, in Verbindung mit Werkstoffkontakt, stellt, wie vielfach gezeigt, die nachteiligste Umgebung für Insulin dar. Dies ist bei konventionellen Lösungen ein praktisches Problem, da vom Hersteller evtl. entgaste Lösungen durch Bewegung (z. B. Transport) und Diffusion letztlich wieder Luft lösen. Demgegenüber unterliegt ein erstarrtes Gel, das als Flüssigkeit entgast wurde, viel weniger diesem Einfluß; auf ein umständliches Entgasen (und der damit verbundenen Gefahr von Unsterilitäten) direkt vor der Anwendung in der Pumpe kann dann möglicherweise verzichtet werden.

Ein weiterer praktischer Vorteil der erfindungsgemäßen Zubereitungen kann darin bestehen, daß, falls es sich um bei der Lagertemperatur erstarrte Gele handelt, ein direkter Kontakt mit dem Stopfen vermieden wird. Die üblichen Stopfen neigen nämlich z. B. zur Absorption der Stabilisatoren, was in Anbetracht der teilweise geringen Mengen problematisch sein kann.

Zur weiteren Erläuterung sollen die folgenden Beispiele dienen, ohne daß die Erfindung auf diese eingeschränkt wäre. Die darin hergestellten Zubereitungen haben bei 4°C sämtlich eine Viskosität von über 1,75 mPa.s. Die Ziffern bei Dextran, z. B. 60, geben, mit $10^3$ multipliziert, das Molgewicht an. Das destillierte Wasser war jeweils p.i. vom pH 7,3. Polygeline ist ein Produkt der Behringwerke AG, Marburg.

**Beispiel 1 und 2,** *Insulin-Zubereitungen mit 20 % Polygeline*

Gemäß *Beispiel 1* wurden unter sterilen Bedingungen vereinigt je eine sterile Lösung von

a) 250 g Polygeline, lyophylisiert, in dest. Wasser, auf 1 l aufgefüllt, und
b) 4,464 g Humaninsulin (28 I.E/mg), 21,25 g Glycerin, 7,50 g Tris-(hydroxymethyl)-aminomethan, 3,375 g Phenol, so viel wasserfreiem Zinkchlorid, daß der Gesamt-Zink-Gehalt 0,035 g betrug, und 0,0125 g Polypropylenglykol, dem beidseitig jeweils etwa 5 % Polyäthylenglykol anpolymerisiert worden war (mittleres Molekulargewicht 1800), in dest. Wasser auf 250 ml aufgefüllt.

Gemäß *Beispiel 2* wurden unter sterilen Bedingungen vereinigt je eine sterile Lösung von

a) 200 g Polygeline, in dest. Wasser, auf 800 ml aufgefüllt, und von
b) 1,429 g Humaninsulin (28 I.E./mg), 1,50 g m-Kresol, 1,00 g Phenol, 17,00 g Glycerin, so viel wasserfreiem Zinkchlorid, daß der Gesamt-Zink-Gehalt 0,028 g betrug und 0,030 g Lecithin, in dest. Wasser, auf 200 ml aufgefüllt.

Die gemäß Beispielen 1 und 2 hergestellten Lösungen werden in üblicher Weise in Glasfläschchen abgefüllt. Bei ca. 15°C erstarrten sie als klare Gele mit 100 I.E./ml.

**Beispiele 3 bis 7):** *Insulinzubereitungen mit 8 bzw. 16 % Dextran*

Nach *Beispielen 3 und 4* wurden unter sterilen Bedingungen vereinigt je eine sterile Lösung von

a) 180 g Dextran 60 bzw. 160 g Dextran 60, in dest. Wasser auf 800 ml aufgefüllt, und von
b) 3,571 g Humaninsulin (28 I.E./mg), 6,00 g Tris-(hydroxymethyl)-aminomethan, 2,00 g m-Kresol, 1,00 g Phenol, 17,00 g Glycerin und so viel wasserfreiem Zinkchlorid, daß der Gesamt-Zink-Gehalt 0,028 g betrug, und 0,010 g Polypropylenglykol (s. Beispiel 1) in dest. Wasser, auf 200 ml aufgefüllt.

Diese Zubereitungen wurde in üblicher Weise in Glasfläschchen abgefüllt.

Nach *Beispielen 5 bis 7* wurden analog Beispiel 3 Insulinzubereitungen, jedoch mit 10 bzw. 20 % Dextran unterschiedlichen Molekulargewichts hergestellt (s. Tabelle 1).

**Beispiel 8)** *Proinsulinzubereitung mit 20 % Polygeline*

Unter sterilen Bedingungen wurden vereinigt je eine sterile Lösung von

a) 20 g Polygeline in dest. Wasser, auf 80 ml aufgefüllt, und von
b) 100 mg Proinsulin vom Schwein, 0,21 g $NaH_2PO_4 \cdot 2H_2O$, 0,30 g m-Kresol, so viel wasserfreiem Zinkchlorid, daß der Gesamt-Zink-Gehalt 0,0012 g betrug, und 0,010 g Polyoxyäthylen-23-laurylläther vom Molekulargewicht 1200 in dest. Wasser auf 20 ml aufgefüllt.

**Physikalische Stabilität der Zubereitungen gemäß Beispielen 1 und 3 bis 7**

In einem standardisierten Pumpversuch durch eine Peristaltikpumpe (37°C, Bewegung, Pumprate 12 I.E./d) wurden folgende Stabilitätsdaten erhalten:

**Tabelle 1**

| Lösung | Zeitraum bis zur ersten Trübung | relative Stabilität |
|---|---|---|
| H-Insulin Hoechst® | 3 Tage | 1 |
| Zubereitung b) nach Beispiel 1, mit dest. Wasser auf 1,25 l aufgefüllt | 45 Tage | 15 |
| Zubereitung nach Beispiel 1 | > 80 Tage | > 27 |
| Zubereitung mit 8 % Dextran (Beispiel 3) | > 60 Tage | > 20 |
| Zubereitung mit 16 % Dextran (Beispiel 4) | > 80 Tage | > 27 |
| Zubereitung mit 10 % Dextran 32 (Beispiel 5) | > 60 Tage | > 20 |
| Zubereitung mit 10 % Dextran 100 (Beispiel 6) | > 60 Tage | > 20 |
| Zubereitung mit 20 % Dextran 100 (Beispiel 7) | > 80 Tage | > 27 |

**Physikalische Stabilität der Zubereitungen gemäß Beispielen 2 und 8**

Die hergestellten Lösungen wurden in einem standardisierten Umpumpversuch durch eine Peristaltikpumpe (37°C, Bewegung, rezyklisierendes Pumpen mit einer Rate von 5 ml/h= 500 I.E./h= 12000 I.E./d) auf ihre physikalische Stabilität untersucht.

**Tabelle 2**

| Lösung | Zeitraum bis zur ersten Trübung | relative Stabilität |
|---|---|---|
| H-Insulin Hoechst® | 20 h | 1 |
| Zubereitung mit 20 % Polygeline (Beispiel 2) | > 7 Tage | > 8 |
| Zubereitung gemäß Beispiel 2, Lösung b)* | 2 Tage | 2,4 |
| Zubereitung mit 20 % Polygeline (Beispiel 8) | > 7 Tage | > 8 |
| Zubereitung gemäß Beispiel 8, Lösung b)* | 30 h | 1,5 |

* jeweils ohne Polygeline, jedoch auf 1 l (Beispiel 2) bzw. 100 ml (Beispiel 8) mit dest. Wasser aufgefüllt.

**Beispiel 9 und 10:** *Physikalische Stabilität von Zubereitungen, die nur Verdickungsmittel enthalten*

Gemäß *Beispiel 9* wurde eine Zubereitung verwendet, die gemäß Beispiel 1 hergestellt wurde, jedoch mit der Abweichung, daß der Lösung b) kein Polypropylenglykol zugesetz war.

Gemäß *Beispiel 10* wurden unter sterilen Bedingungen vereinigt je eine sterile Lösung von

a) 1 l einer 10 %-igen Lösung von Polygeline und
b) 1,786 g Schweineinsulin (28 I.E./mg) und 4,25 g Glycerin, 1,50 g Tris-(hydroxymethyl)-aminomethan, 2,50 g Phenol und so viel wasserfreiem Zinkchlorid, daß der Gesamt-Zink-Gehalt 0,014 g betrug, in dest. Wasser, auf 250 ml aufgefüllt.

Die so hergestellte Zubereitung enthielt nach biologischem Test 40 I.E./ml.

In einem standardisierten Rotationsversuch bei 37°C, 1 Hz werden jeweils 5 Fläschchen einer Zubereitung nach Beispiel 9 und 10 getestet. Zum Vergleich wurde ein Standardinsulin untersucht.

**Tabelle 3**

| Lösung | Zeitraum bis zur ersten Trübung | relative Stabilität |
|---|---|---|
| H-Insulin Hoechst® | 2 Tage | 1 |
| Zubereitung b) nach Beispiel 10, mit dest. Wasser auf 1,25 l aufgefüllt | 4 Tage | 2 |
| Zubereitung nach Beispiel 9 | 15 Tage | 7,5 |
| Zubereitung nach Beispiel 10 | 22 Tage | 11 |

**Wirkprofil von Insulinzubereitungen gemäß Beispiel 1**

i.v. Applikation am Hund und am Kaninchen

Es wurden von der Zubereitung gemäß Beispiel 1 jeweils 0,2 I.E./kg Körpergewicht in die Ohrvene appliziert. Als Vergleichsinsulin diente Humaninsulin Hoechst® (II). Fig. 1 und 2 zeigen den zeitlichen Verlauf des Blutglucosespiegels (x ± SEM). Die Werte wurden aus Messungen an jeweils 5 Tieren ermittelt. Die Zubereitung gemäß Beispiel 1 (I) war sowohl beim Hund (Fig. 1) als auch beim Kaninchen (Fig. 2) so schnell oder sogar eher noch schneller wirksam als der Vergleich Altinsulin (II).

In den Figuren 1 und 2 deutet die Länge der Abstriche bei den einzelnen Meßwerten in üblicher Weise die Standardabweichung vom Mittelwert (SEM) an.

**Patentansprüche** für die Vertragsstaaten: BE, CH, DE, FR, GB, IT, LI, LU, NL, SE.

1. Wäßrige Insulinzubereitung mit einer Insulinkonzentration über 1 I.E./ml und einem Gehalt an stabilisierenden physiologisch verträglichen Mitteln, dadurch gekennzeichnet, daß sie bei 4°C eine Viskosität von mindestens 1,75 mPa.s hat und als stabilisierende physiologisch verträgliche Mittel eine Kombination

A) eines physiologisch verträglichen Verdickungsmittels der Art Polygeline und/oder Dextran mit
B) einer physiologisch verträglichen oberflächenaktiven Substanz aus der Gruppe
   B1) Äthylenglycol-Propylenglycol-Mischpolymerisat oder -Blockpolymerisat wobei jedoch mindestens die Hälfte der Kettenglieder die Einheiten des 1,2-Propylenglycols sind;
   B2) Lecithin
   B3) Polyoxyäthylen-23-lauryläther

enthält.

2. Mittel gemäß Anspruch 1, gekennzeichnet durch wenigstens eines der Merkmale,
   a) daß die Insulinkonzentration bis 1500, vorzugsweise zwischen 5 und 1000 I.E./ml liegt,
   b) daß das Insulin Humaninsulin, ein Säugerinsulin, ein modifiziertes Insulin oder ein humanes Proinsulin ist,
   c) daß es mehr als 1, vorzugsweise 2 bis 20 Gew.-% Verdickungsmittel enthält,

d) daß es als Verdickungsmittel Polygeline enthält,
e) daß es einen pH-Wert zwischen 2,5 und 8,5, vorzugsweise zwischen 6 und 8, aufweist und
f) daß es bei einer Temperatur von 4°C als Hydrogel vorliegt.

3. Mittel gemäß Anspruch 1 oder 2, dadurch gekennzeichnet, daß es
a) ein geeignetes Isotoniemittel,
b) ein geeignetes Konservierungsmittel,
c) eine geeignete Puffersubstanz und/oder
d) Zink in einer Menge bis zu 100 µg Zinkionen/100I.E. enthält.

4. Mittel gemäß einem oder mehreren der Ansprüche 1 bis 3, dadurch gekennzeichnet, daß es zusätzlich ein Hilfsmittel mit verzögernder Wirkung enthält.

5. Mittel nach einem oder mehreren der Ansprüche 1 bis 4, dadurch gekennzeichnet, daß die Viskosität der Zubereitung mindestens 2 und insbesondere mindestens 2,5 mPa.s beträgt.

6. Mittel gemäß einem oder mehreren der Ansprüche 1 bis 5, insbesondere in Form einer Injektionslösung zur Anwendung bei der Behandlung von Diabetes mellitus.

7. Verwendung der Kombination

A) eines physiologisch verträglichen Verdickungsmittel der Art Polygeline und/oder Dextran mit:

B) einer physiologisch verträglichen oberflächenaktiven Substanz aus der Gruppe
B1) Äthylenglycol-Propylenglycol-Mischpolymerisat oder -Blockpolymerisat,
wobei jedoch mindestens die Hälfte der Kettenglieder die Einheiten des 1,2-Propylenglycols sind,
B2) Lecithin,
und/oder
B3) Polyoxyäthylen-23-lauryläther

zur Herstellung einer stabilisierten wäßrigen Insulinzubereitung gemäß einem oder mehreren der Ansprüche 1 bis 5.

**Patentansprüche** für den Vertragsstaat: AT

1. Verfahren zur Herstellung einer gegen mechanische Belastungen stabilisierten wäßrigen Insulinzubereitung, die eine Insulinkonzentration über 1 I.E./ml und bei 4°C eine Viskosität von mindestens 1,75 mPa.s hat, dadurch gekennzeichnet, daß man einer wäßrigen Insulinzubereitung

A) ein physiologisch verträgliches Verdickungsmittel der Art Polygeline und/oder Dextran
B) in Kombinationen mit einer physiologisch verträglichen oberflächenaktive Substanz aus der Gruppe
B1) Äthylenglycol-Propylenglycol-Mischpolymerisat oder- Blockpolymerisat, wobei jedoch mindestens die Hälfte der Kettenglieder die Einheiten des 1,2-Propylenglycols sind,
B2) Lecithin
und/oder
B3) Polyoxyäthylen-23-lauryläther

zusetzt.

2. Verfahren gemäß Anspruch 1, gekennzeichnet durch wenigstens eines der Merkmale,

a) daß die Insulinkonzentration bis 1500, vorzugsweise zwischen 5 und 1000 I.E./ml liegt,
b) daß das Insulin Humaninsulin, ein Säugerinsulin, ein modifiziertes Insulin oder ein humanes Proinsulin ist,
c) daß mehr als 1, vorzugsweise 2 bis 20 Gew.-% Verdickungsmittel zugesetzt wird,
d) daß man als Verdickungsmittel Polygeline zusetzt,
e) daß man einen pH-Wert 2,5 und 8,5, vorzugsweise zwischen 6 und 8, einstellt, und
f) daß die Insulinzubereitung bei einer Temperatur von 4°C Hydrogel vorliegt.

3. Verfahren gemäß Anspruch 1 oder 2, dadurch gekennzeichnet,
daß man
a) ein geeignetes Isotoniemittel,
b) ein geeignetes Konservierungsmittel,
c) eine geeignete Puffersubstanz und/oder
d) Zink in einer Menge bis zu 100 µg Zinkionen/100 I.E. zusetzt.

4. Verfahren gemäß einem oder mehreren Ansprüche 1 bis 3, dadurch gekennzeichnet, daß man zusätzlich ein Hilfsmittel mit verzögernder Wirkung zusetzt.

5. Verfahren nach einem oder mehreren der Ansprüche 1 bis 4, dadurch gekennzeichnet, daß man eine Zubereitung der Viskosität mindestens 2 und insbesondere mindestens 2,5 mPa.s herstellt.

6. Verfahren gemäß einem oder mehreren der Ansprüche 1 bis 5, dadurch gekennzeichnet, daß man die Insulinzubereitung insbesondere in Form einer Injektionslösung zur Anwendung bei der Behandlung von Diabetes mellitus herstellt.

7. Verwendung der Kombination

A) eines physiologisch verträglichen Verdickungsmittels der Art Polygeline und/oder Dextran mit
B) einer physiologisch verträglichen oberflächenaktiven Substanz aus der Gruppe
   B1) Äthylenglycol-Propylenglycol-Mischpolymerisat oder -Blockpolymerisat, wobei jedoch mindestens die Hälfte der Kettenglieder die Einheiten des 1,2-Propylenglycols sind,
   B2) Lecthin,
   und/oder
   B3) Polyoxyäthylen-23-lauryläther

zur Herstellung einer stabilisierten wäßrigen Insulinzubereitung gemäß einen oder mehreren der Ansprüche 1 bis 5.

**Claims** for the Contracting States: BE, CH, DE, FR, GB, IT, LI, LU, NL, SE

1. An aqueous insulin formulation with an insulin concentration above 1 I.U./ml and a content of stabilizing physiologically acceptable agents, characterized by having a viscosity of at least 1.75 mPa.s at 4°C and by containing, as stabilizing physiologically acceptable agents, a combination

A) of a physiologically acceptable thickener of the polygeline and/or dextran type with
B) a physiologically acceptable surface-active substance from the group comprising
   B1) an ethylene glycol/propylene glycol copolymer or block polymer, but at least half of the chain members being units of 1,2-propylene glycol;
   B2) lecithin
   and/or
   B3) polyoxyethylene 23-lauryl ether.

2. An agent as claimed in claim 1, characterized by at least one of the features that
   a) the insulin concentration is up to 1,500, preferably between 5 and 1,000 I.U./ml,
   b) that the insulin is human insulin, a mammalian insulin, a modified insulin or a human proinsulin,
   c) that it contains more than 1, preferably 2 to 20 % by weight of thickener,
   d) that it contains polygeline as the thickener,
   e) that it has a pH of between 2.5 and 8.5, preferably between 6 and 8, and
   f) that it is in the form of a hydrogel at a temperature of 4°C.

3. An agent as claimed in any one of claims 1 and 2, characterized in that it contains
   a) a suitable isotonicizing agent,
   b) a suitable preservative,
   c) a suitable buffer substance and/or
   d) zinc in an amount of up to 100 µg of zinc ions/100 I.U.

4. An agent as claimed in any one of claims 1 to 3, characterized in that it additionally contains an auxiliary with a retarding action.

5. A agent as claimed in any one of claims 1 to 4, characterized in that the viscosity of the formulation is at least 2 and in particular at least 2.5 mPa.s.

6. An agent as claimed in any one of claims 1 to 5, in particular in the form of an injection solution, for use in the treatment of Diabetes mellitus.

7. The use of the combination

A) of a physiologically acceptable thickener of the polygeline and/or dextran type with
B) a physiologically acceptable surface-active substance from the group comprising
   B1) an ethylene glycol/propylene glycol copolymer or block polymer, but at least half of the chain members being units of 1,2-propylene glycol,
   B2) lecithin,
   and/or

B3) polyoxyethylene 23-lauryl ether

for the preparation of a stabilized aqueous insulin formulation as claimed in any one of claims 1 to 5.

**Claims** for the Contracting State: AT

1. A process for the preparation of an aqueous insulin formulation which is stabilized against mechanical stresses and which has an insulin concentration above 1 I.U./ml and a viscosity at 4°C of at least 1.75 mPa.s, characterized by adding to an aqueous insulin formulation

A) a physiologically acceptable thickener of the polygeline and/or dextran type in combination with
B) a physiologically acceptable surface-active substance from the group comprising
B1) an ethylene glycol/propylene glycol copolymer or block polymer, but at least half the chain members being units of 1,2-propylene glycol,
B2) lecithin,
and/or
B3) polyoxyethylene 23-lauryl ether

2. The process as claimed in claim 1, characterized by least one of the features that

a) the insulin concentration is up to 1,500, preferably between 5 and 1,000 I.U./ml,
b) the insulin is human insulin, a mammalian insulin, a modified insulin or a human proinsulin,
c) more than 1, preferably 2 to 20 % by weight of thickener are added,
d) polygeline is added as the thickener,
e) a pH of between 2.5 and 8.5, preferably between 6 and 8, is established and
f) the insulin formulation is present as a hydrogel at a temperature of 4°C.

3. The process as claimed in either of claims 1 and 2, characterized by adding
a) a suitable isotonicizing agent,
b) a suitable preservative,
c) a suitable buffer substance and/or
d) zinc in an amount of up to 100 µg of zinc ions/100 I.U.

4. The process as claimed in any one of claims 1 to 3, characterized by adding additionally an auxiliary with a retarding action.

5. The process as claimed in any one of claims 1 to 4, characterized in that a formulation with a viscosity of at least 2 and in particular at least 2.5 mPa.s is prepared.

6. The process as claimed in any one of claims 1 to 5, characterized in that the insulin formulation is prepared, in particular in the form of an injection solution, for use in the treatment of diabetes mellitus.

7. The use of the combination

A) of a physiologically acceptable thickener of the polygeline and/or dextran type with
B) a physiologically acceptable surface-active substance from the group comprising
B1) an ethylene glycol/propylene glycol copolymer or block polymer, but at least half the chain members being units of 1,2-propylene glycol,
B2) lecithin,
and/or
B3) polyoxyethylene 23-lauryl ether,

for the preparation of a stabilized aqueous insulin formulation as claimed in any one of claims 1 to 5.

**Revendications** pour les Etats Contractants: BE, CH, DE, FR, GB, IT, LI, LU, NL, SE

1. Préparation d'insuline aqueuse ayant une concentration d'insuline supérieure à 1 U.I./ml et contenant des agents stabilisants physiologiquement compatibles, caractérisée en ce qu'elle a une viscosité d'au moins 1,75 mPa.s à 4°C et qu'elle contient comme agents stabilisants physiologiquement compatibles une combinaison:

A) d'un agent épaississant physiologiquement compatible du type Polygèline et/ou Dextran avec
B) une substance tensio-active physiologiquement compatible du groupe
B1) copolymère ou polymère séquencé éthylèneglycol - propylèneglycol dans lequel toutefois la moitié au moins des

éléments de la chaîne sont les motifs du 1,2-propylèneglycol;
B2) la lécithine,
et/ou
B3) le polyoxyéthylène-23-lauryléther.

2. Médicament selon la revendication 1, caractérisé par au moins une des caractéristiques:

a) la concentration de l'insuline peut atteindre 1500, de préférence elle est comprise entre 5 et 1000 U.I./ml,
b) l'insuline est de l'insuline humaine, une insuline de mammifère, une insuline modifiée ou une proinsuline humaine,
c) il contient plus de 1, de préférence 2 à 20 % en poids d'agent épaississant,
d) il contient comme agent épaississant de la Polygèline,
e) il a un pH compris entre 2,5 et 8,5, de préférence entre 6 et 8, et
f) à une température de 4°C, il se présente sous forme d'hydrogel.

3. Médicament selon la revendication 1 ou 2, caractérisé en ce qu'il comprend:

a) un agent d'isotonie approprié,
b) un agent de conservation approprié,
c) une substance tampon appropriée, et/ou
d) du zinc en quantité allant jusqu'à 100 µg d'ions zinc pour 100 U.I.

4. Médicament selon une ou plusieurs des revendications 1 à 3, caractérisé en ce qu'il contient en outre un adjuvant à effet retardateur.

5. Médicament selon une ou plusieurs des revendications 1 à 4, caractérisé en ce que la viscosité de la préparation est d'au moins 2 et en particulier d'au moins 2,5 mPa.s.

6. Médicaments selon une ou plusieurs des revendications 1 à 5, en particulier sous la forme d'une solution pour injection, destiné à l'emploi dans le traitement du diabète sucré.

7. Emploi de la combinaison

A) d'un agent épaississant physiologiquement compatible du type Polygèline et/ou Dextran avec
B) une substance tensio-active physiologiquement compatible du groupe
B1) copolymère ou polymère séquencé éthylèneglycol-propylèneglycol, dans lequel toutefois la moitié au moins des éléments de la chaîne sont les motifs du 1,2-propylèneglycol;
B2) la lécithine,
et/ou
B3) le polyoxyéthylène-23-lauryléther,

pour la fabrication d'une préparation aqueuse d'insuline stabilisée selon une ou plusieurs des revendications 1 à 5.

**Revendications** pour l'Etat Contractant: AT

1. Procédé de fabrication d'une préparation aqueuse d'insuline stabilisée vis-à-vis des sollicitations mécaniques, qui a une concentration d'insuline supérieure à 1 U.I./ml et une viscosité d'au moins 1,75 mPa.s à 4°C, caractérisé en ce que l'on ajoute à une préparation aqueuse d'insuline:

A) un agent épaississant physiologiquement compatible du type Polygèline et/ou Dextran en combinaison avec
B) une substance tensio-active physiologiquement compatible du groupe
B1) copolymère ou polymère séquencé éthylèneglycol-propylèneglycol, dans lequel toutefois au moins la moitié des éléments de la chaîne sont les motifs du 1,2-propylèneglycol,
B2) lécithine;
et/ou
B3) polyoxyéthylène-23-lauryléther.

2. Procédé selon la revendication 1, caractérisé par au moins une des caractéristiques:

a) la concentration de l'insuline peut atteindre 1500, de préférence elle est comprise entre 5 et 1000 U.I./ml,
b) l'insuline est de l'insuline humaine, une insuline de mammifère, une insuline modifiée ou une proinsuline humaine,
c) on ajoute plus de 1, de préférence 2 à 20 % en poids d'agent épaississant,
d) on ajoute comme agent épaississant de la Polygèline,
e) on ajuste le pH à une valeur comprise entre 2,5 et 8,5, de préférence entre 6 et 8; et
f) à une température de 4°C, la préparation d'insuline se présente sous forme d'hydrogel.

3. Procédé selon la revendication 1 ou 2, caractérisé en ce que l'on ajoute:

a) un agent d'isotonie approprié,
b) un agent de conservation approprié,
c) une substance tampon appropriée; et/ou
d) du zinc en quantité allant jusqu'à 100 µg d'ions zinc pour 100 U.I.

4. Procédé selon une ou plusieurs des revendications 1 à 3, caractérisé en ce que l'on ajoute en outre un adjuvant à effet retardateur.

5. Procédé selon une ou plusieurs des revendications 1 à 4, caractérisé en ce que l'on fabrique une préparation ayant une viscosité d'au moins 2 et, en particulier, d'au moins 2,5 mPa.s.

6. Procédé selon une ou plusieurs des revendications 1 à 5, caractérisé en ce que l'on fabrique une préparation d'insuline en particulier sous la forme d'une solution pour injection destinée à l'emploi dans le traitement du diabète sucré.

7. Emploi de la combinaison

A) d'un agent épaississant physiologiquement compatible du type Polygèline et/ou Dextran avec
B) une substance tensio-active physiologiquement compatible du groupe
B1) copolymère ou polymère séquencé éthylèneglycol-propylèneglycol, dans lequel cependant au moins la moitié des éléments de la chaîne sont les motifs du 1,2-propylèneglycol,
B2) lécithine,
et/ou
B3) polyoxyéthylène-23-lauryléther, pour la fabrication d'une préparation d'insuline aqueuse stabilisée selon une ou plusieurs des revendications 1 à 5.

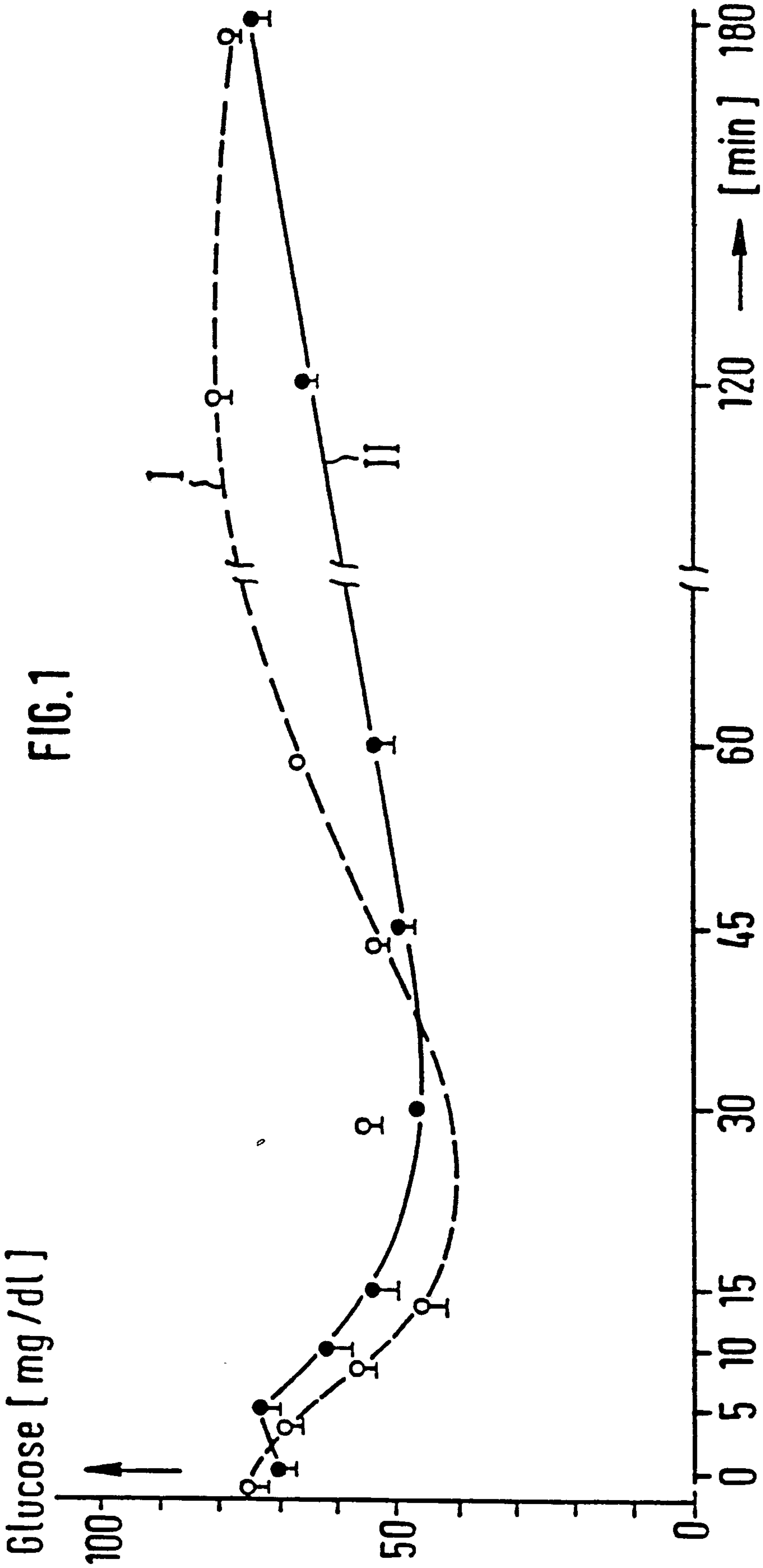


FIG.1

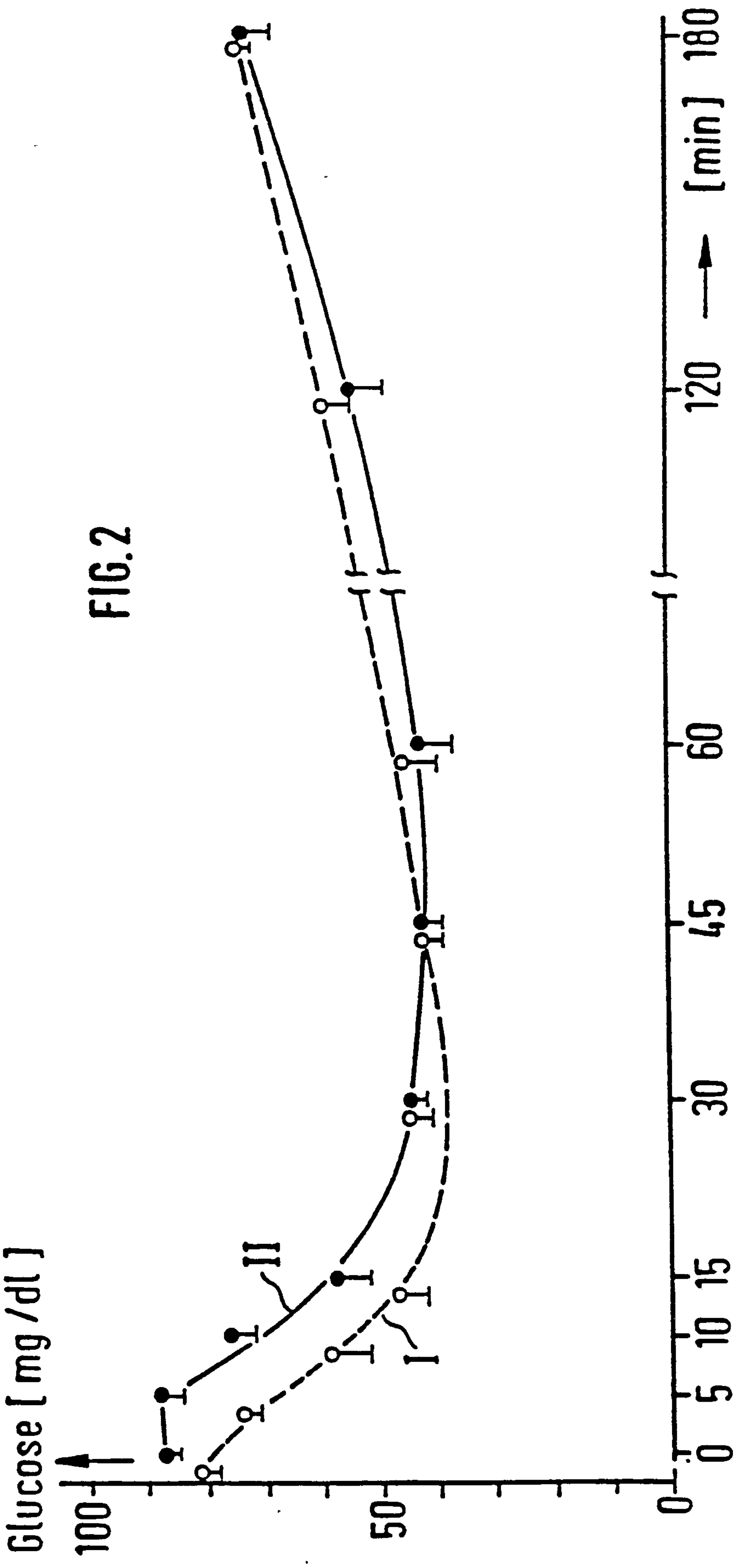


FIG.2